# EUROPEAN PATENT APPLICATION

(11) **EP 4 702 914 A2**
(43) Date of publication of application: **04.03.2026**
(21) Application number: 25197983.7
(22) Date of filing: 25.08.2025
(51) Int. Cl.: A61B 1/05, A61B 1/06

(54) **A SURGICAL INSTRUMENT FOR ENHANCED VISUALIZATION IN MINIMALLY INVASIVE SURGERY AND A SURGICAL SYSTEM**

(30) Priority: 26.08.2024 US 202463687258 P; 26.09.2024 US 202418897046
(71) Applicant: Robotron Surgical IP Holding LLC, Miami, FL 33133 (US)
(72) Inventor: SIEMIONOW, Krzysztof, Chicago, IL 60610 (US)
(74) Representative: Kancelaria Eupatent.pl Sp. z o.o.

(57) **Abstract**

A surgical instrument (10) comprising: a body (11); and a vision module (20) combined with the body (11), the vision module (20) comprising: a camera (22) with a field of view (3) covering a vicinity of a distal end (13) of the surgical instrument (10); and a lighting (23) directed towards the field of view (3).

## Description

### FIELD OF THE INVENTION

The present invention relates to the field of surgery, specifically to minimally invasive procedures including ENT (Ear, Nose, and Throat), neurosurgery, spine surgery, orthopedic surgery interventional radiology, and pain management surgery. The invention integrates advanced imaging, and optionally real-time motion tracking technologies, into surgical instruments to enhance the precision and safety of minimally invasive surgeries.

### BACKGROUND

Minimally invasive surgery (MIS) has revolutionized the field of surgery by reducing recovery time, minimizing scarring, and improving patient outcomes. However, these procedures require high precision, which is often limited by the surgeon's ability to visualize the surgical field and accurately track instrument positioning.

Traditional imaging and tracking systems may provide adequate data but often lack the integration necessary for real-time, intelligent decision-making during surgery. Furthermore, situational awareness is currently limited as imaging data are not correlated with positional data.

### SUMMARY OF THE INVENTION

The present invention addresses the limitations of current surgical instruments by combining a digital camera and optionally other elements, such as an electromagnetic or optical motion tracking sensor, into a single surgical instrument.

The combination may be achieved by providing a new design of the surgical instrument or by attaching the camera onto an existing surgical instrument, such as a Woodson dissector or Penfield dissector. The attachment can be achieved through various methods, including but not limited to, screw-on mechanisms, snap-fit designs, adhesive bonding, or magnetic couplings. The integration can also be achieved by embedding the vision module within the body of the surgical instrument during the manufacturing process, ensuring a seamless and robust connection.

The surgical instrument is the primary tool used in minimally invasive procedures. It can have various surgical forms such as a 90° dissector, 90° ball probe, nerve hook, curved scissors, pituitary rongeur with tooth, straight and upbiting pituitary rongeur, downbiting pituitary rongeur, Woodson dissector, Penfield dissector, suction tube (eg. Frazier styled suction), etc. This versatility allows the instrument to be used across different surgical specialties.

The body of the surgical instrument forms its main structural component, extending from the proximal end to the distal end. It can be made from various materials such as stainless steel, titanium, high-strength polymers, carbon fiber composites, or biocompatible ceramics to ensure durability and sterility. The body can be designed to integrate seamlessly with the vision module or to accommodate it as an attachment.

The proximal end is the part of the instrument that the surgeon holds or that is attached to an articulated arm of a robotic system. It can include ergonomic features such as textured grips or finger rests to enhance control and comfort during surgery. In some embodiments, the proximal end may house components like a controller, battery, and wireless communication module. The ergonomic features can be further specified to include anti-slip coatings, customizable grip sizes, or integrated control buttons for adjusting the vision module settings.

The distal end is the operational section of the instrument that interacts with the surgical site. The distal end can be configured in various shapes and sizes depending on the specific surgical application. Specific configurations can include tapered tips for fine dissection, blunt ends for tissue manipulation, or serrated edges for cutting.

The vision module is a critical component that provides high-resolution visual data of the surgical field. It is located at the distal end and can be integrated into the body or attached as a separate module. The vision module includes several sub-components, each contributing to its functionality.

The housing of the vision module encases the components of the vision module. It can be made from biocompatible materials and designed to be either integrated with the body or attached as a separate unit. Suitable biocompatible materials include medical-grade plastics, titanium, or stainless steel.

The camera captures high-resolution still images or videos of the surgical site in the vicinity of the distal end of the surgical instrument, i.e., in the area immediately surrounding the distal end of the surgical instrument. This includes the surgical site and any anatomical structures within the operational range of the instrument's distal end, typically within a few millimeters to a few centimeters, depending on the specific surgical application and the configuration of the vision module. The field of view of the camera may be set up to encompass a fragment of the distal end or just the area in close proximity to the distal end (such as 1 mm further from the distal end), depending on the needs of the procedure performed. The camera can be a high-resolution digital camera, such as a CCD or CMOS sensor, capable of capturing detailed images or video. The size of the camera can be in the order of 1x1 mm. The camera can be equipped with adjustable focus and field of view settings to cater to different surgical needs. In some embodiments, the camera may include features like optical zoom or image stabilization to enhance visual clarity. Additional features such as infrared imaging or fluorescence imaging can be included for specific surgical applications.

The lighting provides illumination to the surgical field, ensuring that the camera captures clear images. The lighting can comprise a light-emitting diode (LED) or a plurality of LEDs that can be positioned around the camera and may include adjustable brightness settings. The size of the LEDs can be as small as 0,2 x 0,2 mm. The LEDs can emit different color temperatures or wavelengths, such as UV or IR, to enhance visibility of specific tissues or structures.

A separator can be provided between the camera and the lighting to prevent light bleeding into the camera and to avoid interference.

The controller manages the operation of the vision module, including the camera and LEDs. It can be located within the housing or at the proximal end. The controller may act as an interface between the data processing module and the components of the vision module and may perform actions such as image pre-processing and data compression. The controller can also include advanced features such as real-time image enhancement algorithms or automatic exposure control.

The battery provides power to the components of the vision module. It can be integrated into the housing or located at the proximal end of the instrument. The battery may support wireless charging and should offer sufficient capacity to last through lengthy surgical procedures.

The motion tracking indicator tracks the location of the distal end within the surgical field. It can use technologies like electromagnetic or optical motion tracking. The motion tracking indicator provides real-time positional and orientational data that can be used to generate additional information at the data processing module.

The communication module enables data transmission between the vision module and external devices like a data processing module. It can be a wired module, such as a USB interface, or a wireless module that supports various communication protocols such as Wi-Fi, Bluetooth, or proprietary wireless standards.

The components of the vision module, when not housed together, can be connected via a cable that can be routed within the body or along the outer periphery of the body within a dedicated duct. The cable ensures reliable power and/or data transmission between the components. The dedicated duct can be a channel within the body or outside the body that houses the cable. It protects the cable from damage and ensures that it does not interfere with the surgical procedure. The duct can be designed to be easily accessible for maintenance or replacement of the cable.

The surgical instrument can be used in a surgical system in connection with a data processing module responsible for handling and analyzing the data received from the vision module. The data processing module processes visual and positional data to provide real-time feedback to the surgeon. It can perform tasks such as image enhancement, data fusion, and real-time analytics.

The data processing module can be a standalone unit, such as a dedicated computer system located in the operating room. It can also be an integrated system, embedded within a surgical robot or other medical equipment. Furthermore, it can be implemented in a cloud-based environment, wherein data is transmitted to a remote server for processing and then sent back to the operating room.

The data processing module communicates with the vision module of the surgical instrument via a wired or wireless communication interface.

A data processor is the core computational unit within the data processing module. It executes algorithms for image processing, data analysis, and system control. It can be implemented as standard CPUs or GPUs capable of handling complex computations, or specialized FPGAs or ASICs designed for specific tasks like real-time image processing.

An artificial intelligence (AI) module enhances the capabilities of the data processor by adding advanced data analysis features. It performs tasks such as anatomy recognition, segmentation, and annotation using specific algorithms and methods. For anatomy recognition, the AI module may utilize convolutional neural networks (CNNs) trained on large datasets of medical images. Segmentation can be achieved through techniques such as U-Net architectures or region-based convolutional neural networks (R-CNNs), which delineate anatomical structures from the background. Annotation involves labeling the identified structures using pre-trained models. These methods enable the AI module to provide predictive analytics and decision support, enhancing the surgeon's ability to make informed decisions during the procedure.

Visual feedback to the surgeon is provided via an external display, showing real-time images and data overlays. It can display high-resolution images and augmented reality overlays to enhance situational awareness. The display may take the form of a standard medical-grade monitor, or a wearable head-up display integrated into surgical goggles.

The surgeon can interact with the system via a user interface, adjusting settings of the vision module. It allows control of the vision module and the data processing module, including camera settings, lighting adjustments, and data overlays. For example, the user interface may take the form of a keyboard, a pointer, touch control, hands-free operation using voice commands, or tactile feedback for precise control. Some user interface elements can be integrated with the body of the surgical instrument. The user interface can also include customizable presets for different surgical procedures or user preferences.

External data sources may provide additional data that can be integrated into the system for enhanced functionality, such as preoperative imaging data (e.g., MRI or CT scans), patient records, and other relevant information to the data processing module.

Additional information may be generated based on the position signal from the motion tracking indicator, which allows accurate tracking of the instrument's movement within the surgical field. This additional information can be overlaid on preoperative or intraoperative imaging to always inform the surgeon of their position relative to critical anatomical structures, allowing for increased safety and precision of the surgical procedure, leading to better outcomes. The display may be an augmented reality display, for example a head-worn display that allows to overlay the additional information over the real world image as seen by the surgeon. The AI module can recognize and differentiate between various anatomical structures, such as pedicles, nerves, and ligaments. For example, the system can measure the surface area of these structures, providing the surgeon with additional quantitative data to assist in decision-making.

For example, the motion tracking indicator data can be used to create a "painting" effect, where the instrument's contact points on the anatomy are recorded and matched with preoperative MRI or CT scans. The AI system may correlate the real-time camera data with the motion tracking indicator data and preoperative imaging, enhancing the accuracy of the instrument's placement and navigation. This increases the surgeon's situational awareness.

The integration of visual and positional data allows for a more comprehensive understanding of the surgical field. The data from the camera helps refine the positional data from the motion tracking indicator, and vice versa, leading to improved precision in surgical procedures. As a result, there is potential to reduce human error, such as wrong level (or site) surgery, avoid critical neurovascular structures, and ensure that the area of pathology is correctly identified and treated.

The present invention provides a novel and advanced surgical instrument designed to enhance the precision and safety of minimally invasive surgeries. Unlike traditional surgical tools, this invention integrates a vision module with various other components to offer real-time visual and instrument position and orientation to the surgeon.

The object of the present invention represents a synergistic integration where the combined elements produce new and enhanced results. The vision module, which includes a high-resolution camera, lighting, and optionally motion tracking, works in synergy with the surgical instrument to provide real-time visual as well as position and orientation of the surgical instrument. This integration allows for improved situational awareness, precision, and safety during surgery, which is not achievable by the individual components alone. This functionality can be further enhanced by advanced features such as adjustable focus, optical zoom, image stabilization, and real-time data processing through an AI module. These features collectively enhance the surgeon's ability to visualize and navigate the surgical field, reducing the risk of errors and improving patient outcomes.

The surgical system of the present invention allows for real-time fusion of visual and instrument motion tracking data. The vision module's camera and lighting provide high-resolution images, while the tracking system ensures accurate instrument placement. This data is processed and analyzed in real-time by an external data processing module, which can overlay additional information, such as anatomical structures and preoperative imaging, onto the live video feed. This level of integration and real-time data fusion is not found in existing surgical instruments. Furthermore, the inclusion of an AI module within the data processing system adds the ability to perform tasks such as anatomy recognition, segmentation, and annotation, providing the surgeon with critical information during the procedure. This capability enhances decision-making and situational awareness, leading to safer and more effective surgeries.

In a specific aspect, the invention relates to a surgical instrument comprising: a body; a vision module combined with the body, the vision module comprising: a camera with a field of view covering a vicinity of a distal end of the surgical instrument; and a lighting directed towards the field of view.

Preferably, the field of view covers a fragment of the distal end of the surgical instrument.

In general, the location, angle and field of view of the camera shall be configured such that the distal end of the surgical instrument occupies no more than 50%, or no more than 40%, or no more than 30%, or no more than 20%, or no more than 25%, or no more than 20%, or no more than 15%, or no more than 10%, or no more than 5%, or no more than 3%, or no more than 1% of the field of view.

The distance D between the camera and the end of the tip of the instrument is measured in a straight line from the camera's center to the furthest point of the tip visible within the camera's field of view. It can be selected depending on the type of instrument, its dimensions, the type of the camera, the optical parameters of the lens of the camera, such as the angular field of view. In typical applications, the distance D is usually between 3 and 15 mm.

The angular field of view of the camera can be from very narrow, such as 10 degrees (in specialized applications requiring high focus) even up to very wide, such as 180 degrees or more (fisheye, in specialized applications requiring extremely wide field of view), typically between 60 and 120 degrees, for example 90 degrees.

Preferably, the surgical instrument further comprises a motion tracking indicator configured to indicate the position of the distal end of the surgical instrument.

Preferably, the vision module is contained in a housing integrated with the body of the surgical instrument.

Preferably, the vision module is contained in a housing attached to the body of the surgical instrument.

Preferably, the housing of the vision module comprises a controller.

Preferably, the housing of the vision module comprises a battery for supplying power to components of the vision module.

Preferably, the battery is configured for wireless charging.

Preferably, the housing of the vision module comprises a communication interface for transmitting signals to and/or from the components of the vision module.

Preferably, the surgical instrument further comprises a controller located at a distance from the housing of the vision module.

Preferably, the surgical instrument further comprises a battery located at a distance from the housing of the vision module.

Preferably, the surgical instrument further comprises a communication interface located at a distance from the housing of the vision module.

Preferably, the surgical instrument body shape is selected from the group consisting of a 90° dissector, a 90° ball probe, a nerve hook, curved scissors, a pituitary with tooth, a straight and upbiting pituitary, a downbiting pituitary, a Woodson dissector, a Penfield instrument and a suction tube.

In another aspect, the invention relates to a surgical system comprising a surgical instrument as described herein and a data processing module comprising a data processor configured to process data received from the camera of the vision module.

Preferably, the data processor comprises an artificial intelligence module for performing at least one of: recognizing, segmenting, and annotating parts of the anatomy visible in the data received from the camera of the vision module.

Preferably, the data processor is further configured to generate additional information to be integrated with the video signal from the camera.

Preferably, the additional information is generated based on data from preoperative MRI or CT scans.

In another aspect, the invention relates to a method for providing enhanced visualization of image data of a surgical field, the method comprising providing a surgical system as described herein; receiving image data from the camera of the vision module; processing the image data by the data processing module to generate a processed image; and displaying the processed image on a display.

These and other features, aspects, and advantages of the invention will become better understood with reference to the following drawings, descriptions, and claims.

### BRIEF DESCRIPTION OF DRAWINGS

Various embodiments are herein described, by way of example only, with reference to the accompanying drawings, wherein:
Fig. 1A shows schematically a first embodiment of a surgical instrument in the form of a 90° dissector.
Fig. 1B shows schematically a second embodiment of the surgical instrument in the form of a 90° ball probe.
Fig. 1C shows schematically a third embodiment of the surgical instrument in the form of a nerve hook.
Fig. 1D shows schematically a fourth embodiment of the surgical instrument in the form of curved scissors.
Fig. 1E shows schematically a fifth embodiment of the surgical instrument in the form of a pituitary rongeur with tooth.
Fig. 1F shows schematically a sixth embodiment of the surgical instrument in the form of a straight and upbiting pituitary rongeur.
Fig. 1G shows schematically a seventh embodiment of the surgical instrument in the form of a downbiting pituitary rongeur.
Fig. 1H shows schematically an eighth embodiment of the surgical instrument in the form of a suction tube (e.g. Frazier styled suction).
Fig. 2A shows a close-up view of an ninth embodiment of the surgical instrument.
Fig. 2B shows a close-up view of a tenth embodiment of the surgical instrument.
Fig. 2C shows a close-up view of a eleventh embodiment of the surgical instrument.
Fig. 2D shows a close-up view of a twelfth embodiment of the surgical instrument.
Fig. 3A shows an example of the use of the surgical instrument.
Fig. 3B shows a first example view of an image obtained from the vision module.
Fig. 3C shows a second example view of an image obtained from the vision module.
Fig. 3D shows a third example view of an image obtained from the vision module.
Fig. 4A shows schematically a first embodiment of coupling the vision module with the body of the surgical instrument.
Fig. 4B shows schematically a second embodiment of coupling the vision module with the body of the surgical instrument.
Fig. 4C shows schematically a third embodiment of coupling the vision module with the body of the surgical instrument.
Fig. 4D shows schematically a fourth embodiment of coupling the vision module with the body of the surgical instrument.
Fig. 5 shows a functional diagram of a surgical system.
Fig. 6 shows steps of a method for providing enhanced visualization of image data of a surgical field.

### DETAILED DESCRIPTION OF EMBODIMENTS

The following detailed description is of the best currently contemplated modes of carrying out the invention. The description is not to be taken in a limiting sense but is made merely for the purpose of illustrating the general principles of the invention.

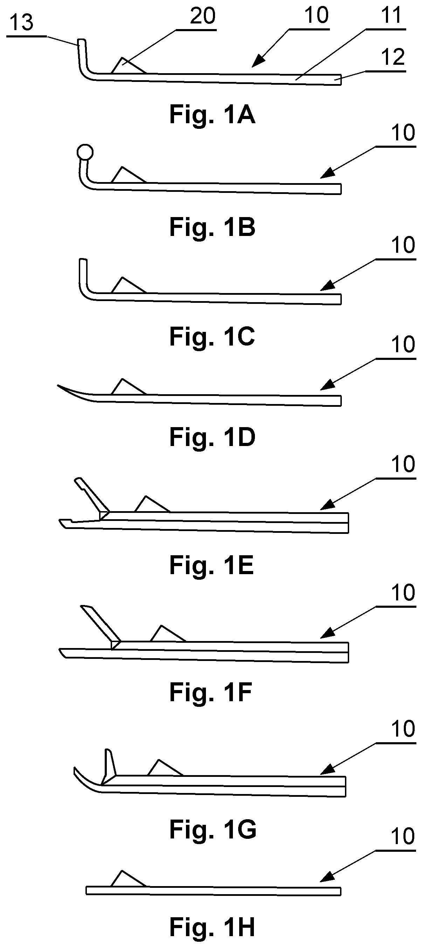

Figs. 1A-1H show schematically side views of eight embodiments of surgical instruments. The surgical instruments 10 have a body 11 with a proximal end 12 and a distal end 13. The body 11 has a form typical to a well-known instrument, such as a 90° dissector (Fig. 1A), a 90° ball probe (Fig. 1B), a nerve hook (Fig. 1C), curved scissors (Fig. 1D), a pituitary rongeur with tooth (Fig. 1E), a straight and upbiting pituitary rongeur (Fig. 1F), a downbiting pituitary rongeur (Fig. 1G) and a suction tube (Fig. 1H). Other instruments, such as a Woodson dissector or Penfield dissector, can be provided in an equivalent manner. A vision module 20 is provided at the distal end 13 of the surgical instrument, with its field of view covering the distal end 13. The vision module 20 is configured to capture a real-time view of the surgical site, as the instrument is directed by the surgeon, which is converted to a video signal that can be displayed on an external display, as will be discussed below.

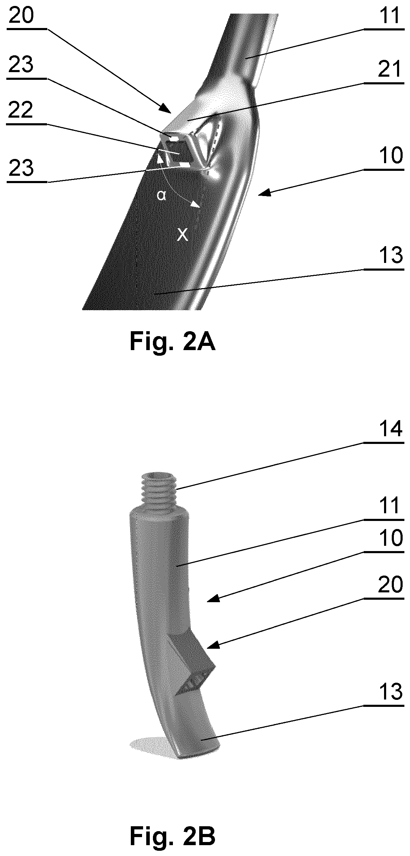

Fig. 2A shows a close-up view of a ninth embodiment of the surgical instrument, illustrating the integration of the vision module 20, which includes a housing 21 integrated with the body 11 of the surgical instrument 10 (i.e., made as a single-piece element). The vision module 20 comprises a camera 22 and a plurality of light-emitting diodes (LEDs) 23 positioned next to the camera 22 to illuminate its field of view, covering the distal end 13 of the surgical instrument 10. The angle α of the camera's 22 line of sight with respect to the longitudinal axis X of the surgical instrument can be adjusted, allowing the surgeon to configure the instrument based on the specific procedure and required field of view.

Fig. 2B shows a close-up view of a tenth embodiment of the surgical instrument 10, designed as a screw-on tool that can be attached via a threaded ending 14 to a handle for manual use by the surgeon or to a robotic arm for automated handling.

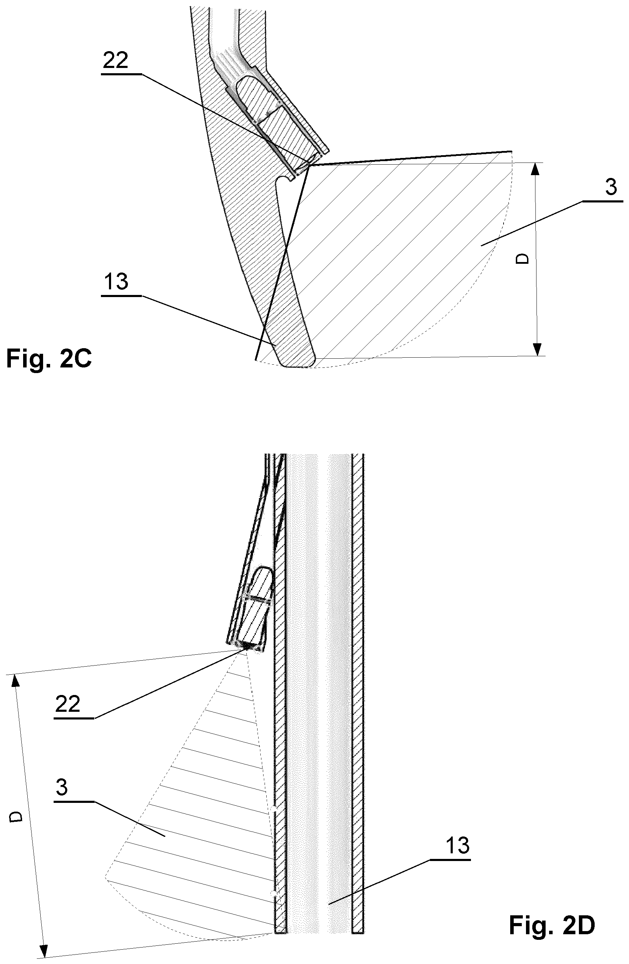

Fig. 2C shows a close-up view of an eleventh embodiment of the surgical instrument 10, in a form of a dissector, with a detailed configuration of the camera's 22 placement and its field of view 3. The camera 22 is placed within a distance D of 6 mm from the tip of the distal end 13 of the instrument 10 and has an angular field of view of about 90 degrees.

Fig. 2D shows a close-up view of a twelfth embodiment of the surgical instrument 10, in a form of a suction tube, with a detailed configuration of the camera's 22 placement and its field of view 3. The camera 22 is placed within a distance D of 12 mm from the tip of the distal end 13 of the instrument 10 and has an angular field of view of about 40 degrees.

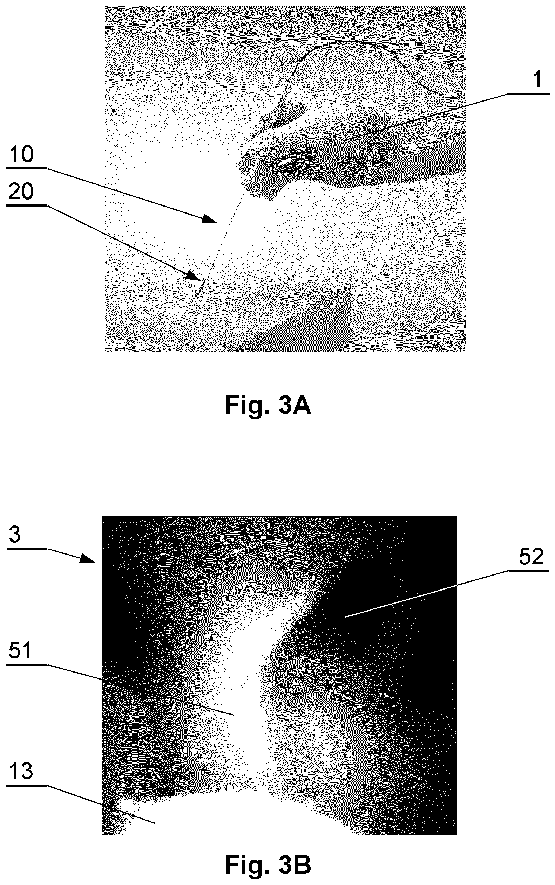

Fig. 3A demonstrates an example of the surgical instrument 10 in use. When a surgeon manipulates the surgical instrument by hand 1, the vision module 20 illuminates the camera's field of view near the distal end 13 of the instrument. This provides an image of the field of view 3 (as shown in Fig. 3B) of the operated area, which can be displayed on an external monitor, offering an enlarged view and visibility of regions that would otherwise be occluded if the instrument operates within body cavities (in this case, related to spine surgery, a pedicle 51 and neural foramina 52 is visible). In Fig. 3B, the distal end 13 of the instrument covers about 8% of the area field of view 3.

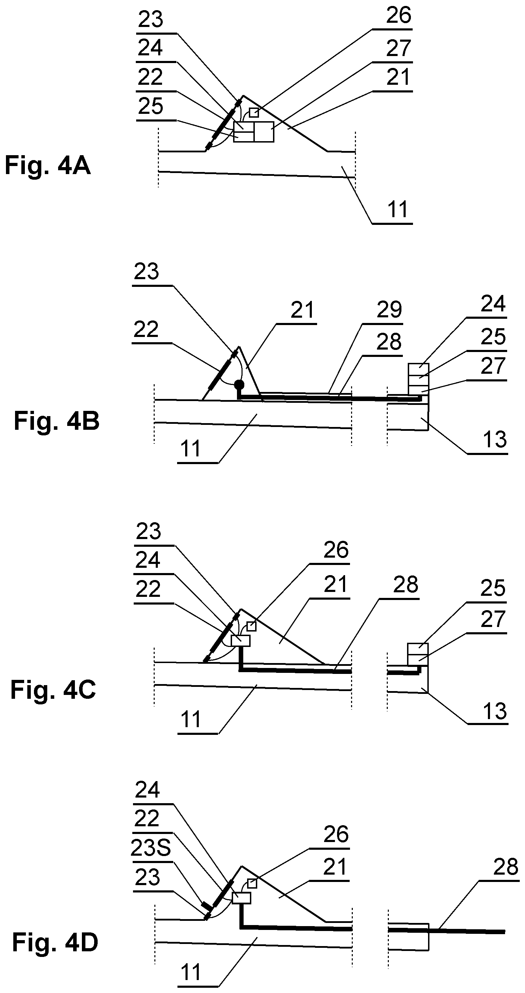

Figs. 4A-4D present various schematic representations of embodiments for coupling the vision module 20 with the body 11 of the surgical instrument.

In the first embodiment shown in Fig. 4A, the housing 21 of the vision module 20 is integrated with the body 11 of the surgical instrument as a single-form element. The housing 21 contains all necessary components for the operation of the vision module 20, including the camera 22, two LEDs 23, a controller 24, a battery 25, a motion tracking indicator 26 (optional), and a wireless communication module 27. The battery 25 can be configured for inductive wireless charging. This design allows the instrument 10 to be used like any other surgical instrument of this type while providing essential video and position data to an external data processing module.

In the second embodiment shown in Fig. 4B, the housing 21 of the vision module 20 is attached to the body 11 of the surgical instrument as an attachment module. The housing 21 contains only the camera 22 and a single LED 23. The controller 24, battery 25, and wireless communication module 27 are positioned away from the housing 21 and located at the proximal end 12 of the surgical instrument 10. Power and data signals are transmitted between the components via a cable 28 that runs along the outer periphery of the surgical instrument in a dedicated duct 29. This design minimizes the size of the housing 21 of the vision module 20.

In the third embodiment shown in Fig. 4C, the housing 21 of the vision module 20 is attached to the body 11 of the surgical instrument as an attachment module. The housing 21 contains the camera 22, a single LED 23, the controller 24, and the motion tracking indicator 26. The battery 25 and wireless communication module 27 are positioned away from the housing 21 and located at the proximal end 12 of the surgical instrument 10. Power and data signals are transmitted between the components via the cable 28 that runs within the body 11 of the surgical instrument.

In the fourth embodiment shown in Fig. 4D, the housing 21 of the vision module 20 is integrated with the body 11 of the surgical instrument. The housing 21 contains the camera 22, two LEDs 23, the controller 24, and the motion tracking indicator 26. A separator 23S is provided between the camera 22 and the LED 23 to prevent light bleeding into the camera 22 and to avoid interference. Power and data signals are transmitted via the cable 28 that runs within the body 11 of the surgical instrument and connects the components within the housing 21 to the external data processing module, which contains the power source.

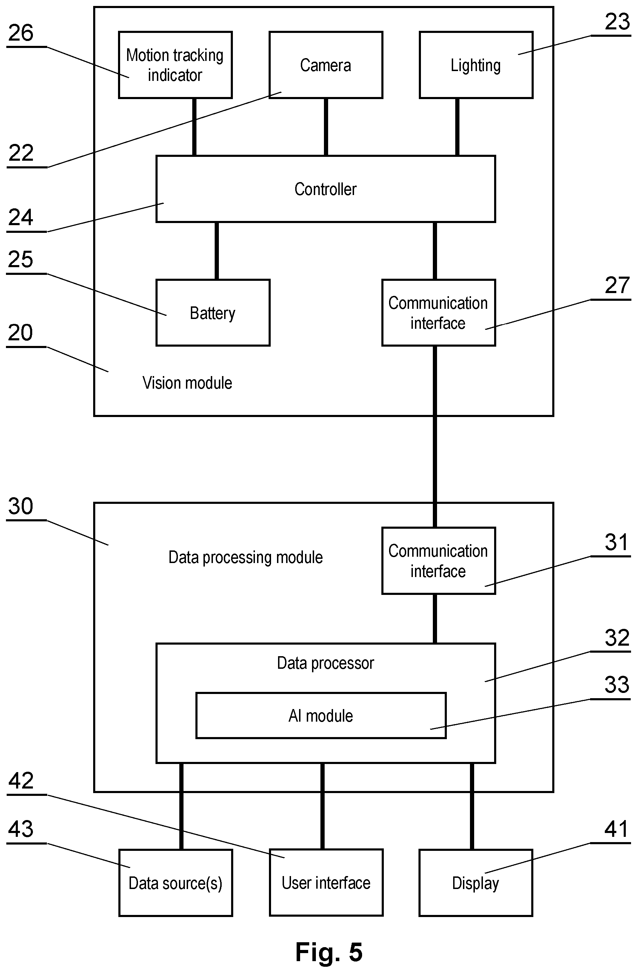

Fig. 5 illustrates a functional diagram of a surgical system comprising a surgical instrument 10 equipped with a vision module 20 that communicates with a data processing module 30. The data processing module 30 includes a communication interface 31 for interacting with the communication interface 27 of the vision module 20 and a data processor 32 for processing data received from the vision module 20. If necessary, the data processor 32 can also send power signals to activate the components of the vision module 20 if it lacks a battery 25 or requires additional power. The data processor 32 is responsible for receiving the video signal from the camera 22 and processing it for display on an external display 41, such as a screen or a head-up display worn by the surgeon.

The data processor 32 can be configured to adjust the area of the total field of view 3 of the camera 22 to the needs of the operator of the device. For example, the camera 22 can cover a wide field of view and the operator may wish to limit it to specific area only. In that case, the data processor can perform simple image cropping, or additional image processing operations such as rescaling to compensate wide field of view distortions (such as fisheye camera distortions), image sharpening etc.

The system can be controlled via a user interface 42, such as a keyboard, pointer, or haptic or voice interface, through which the user may control the operation of the vision module 20. This includes adjusting the field of view of the camera 22 e.g., by changing the angle of view, the field of view or adjusting focus or modifying the intensity of the lighting 23.

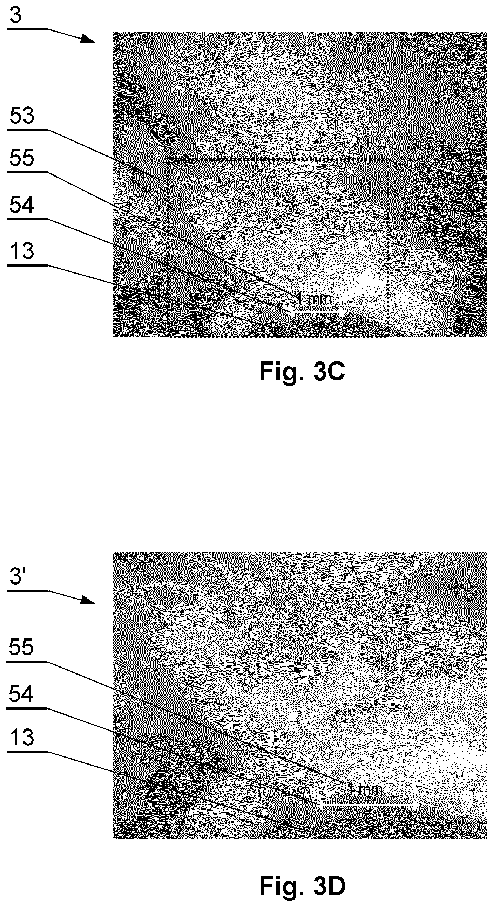

For example, as shown in Fig. 3C, a user may be first provided with a full field of view 3 of the camera 22, and a rectangular overlay box 53 via which the user may select which portion of that full field of view 3 shall be presented. Once the user sets the position and dimensions of the overlay box 53, that limited field of view 3' can be presented (by operation of the data processor 32) in subsequent operation of the device, as shown in Fig. 3D.

Additionally, the data processor 32 may be configured to show a dimensional marker 54 that shows a known dimension, for example 1 mm. That dimensional marker 54 can be generated for example by recognizing a known shape in the image, such as the shape of a portion of a tip of the distal end 13 of the instrument. The dimensional marker 54 can be accompanied by a textual representation 55 of the size. Both the dimensional marker 54 and the textual size representation 55 can be turned on/off as needed.

It may further include dedicated modules, such as an artificial intelligence module 33, for performing additional data processing functions, such as recognizing, segmenting, and annotating visible parts of the anatomy, and generating additional information to be integrated with the video signal from the camera 22. The additional information generated by the artificial intelligence module 33 can be based on data obtained from the vision module 20 and data from external sources 43, such as preoperative MRI or CT scans of the patient.

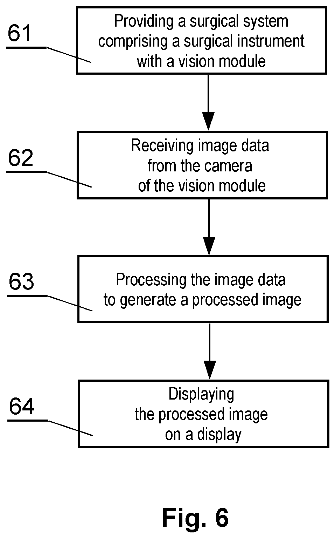

The use of the surgical system as described herein is summarized by the steps of the method shown in Fig. 6, for providing enhanced visualization of image data of a surgical field. In step 61, a surgical system as described herein is provided. Next, in step 62, image data from the camera 22 of the vision module 20 is received by the data processing module 30 and processed in step 63 by the data processing module 30 to generate a processed image of the full field of view 3 or the limited field of view 3', optionally with overlaid additional information, such as the dimensional marker 54. The processed image 3 is then displayed in step 64 on a display 41.

While the invention has been described with respect to a limited number of embodiments, it will be appreciated that many variations, modifications, and other applications of the invention may be made, such as combining the features of different embodiments presented herein or simplifying these embodiments by removing some features that are not essential. Therefore, the claimed invention as recited in the claims that follow is not limited to the embodiments described herein.

## Claims

1. A surgical instrument (10) comprising:
- a body (11); and
- a vision module (20) combined with the body (11), the vision module (20) comprising:
- a camera (22) with a field of view (3) covering a vicinity of a distal end (13) of the surgical instrument (10); and
- a lighting (23) directed towards the field of view (3).

2. The surgical instrument (10) of claim 1, wherein the distal end (13) of the surgical instrument (10) occupies no more than 50%, or no more than 40%, or no more than 30%, or no more than 20%, or no more than 25%, or no more than 20%, or no more than 15%, or no more than 10%, or no more than 5%, or no more than 3%, or no more than 1% of the area of the field of view (3).

3. The surgical instrument (10) of claim 1 or 2, further comprising a motion-tracking indicator (26) configured to indicate the position and orientation of the distal end (13) of the surgical instrument (10).

4. The surgical instrument (10) of any preceding claim, wherein the vision module (20) is contained in a housing (21) integrated with the body (11) of the surgical instrument (10).

5. The surgical instrument (10) of any of claims 1-3, wherein the vision module (20) is contained in a housing (21) attached to the body (11) of the surgical instrument (10).

6. The surgical instrument (10) of claim 4 or 5, wherein the housing (21) of the vision module (20) comprises a controller (24).

7. The surgical instrument (10) of any preceding claim, further comprising a controller (24) located at a distance from the housing (21) of the vision module (20).

8. The surgical instrument (10) of any preceding claim, wherein the surgical instrument body (11) shape is selected from the group consisting of: a 90 ° dissector, a 90 ° ball probe, a nerve hook, curved scissors, a pituitary rongeur with tooth, a straight and upbiting pituitary rongeur, a downbiting pituitary rongeur, a Woodson dissector, a Penfield instrument and a suction tube.

9. A surgical system comprising:
- a surgical instrument (10) according to any of previous claims; and
- a data processing module (30) comprising a data processor (32) configured to process data received from the camera (22) of the vision module (20).

10. The surgical system of claim 9, wherein the data processor (32) comprises an artificial-intelligence module (33) for performing at least one of: recognizing, segmenting and annotating parts of the anatomy visible in the data received from the camera (22) of the vision module (20).

11. The surgical system of claim 9 or 10, wherein the data processor (32) is further configured to generate additional information to be integrated with the video signal from the camera (22).

12. The surgical system of claim 11, wherein the additional information comprises dimensional information (54, 55).

13. The surgical system of claim 11 or 12, wherein the additional information is generated based on data from preoperative MRI or CT scans (43).

14. A method for providing enhanced visualization of image data of a surgical field, the method comprising:
- providing a surgical system according to any of claims 9-13;
- receiving image data from the camera (22) of the vision module (20);
- processing the image data by the data processing module (30) to generate a processed image; and
- displaying the processed image on a display (41).

15. The method of claim 14, further comprising processing the image data so as to limit the field of view (3) of the data received from the camera (22) of the vision module (20) and displaying, as the processed image, the limited field of view (3').
